(19) Europäisches Patentamt
European Patent Office
Office européen des brevets

(11) Numéro de publication: 0 056 548 B2

(12) **NOUVEAU FASCICULE DE BREVET EUROPEEN**

(45) Date de publication du nouveau fascicule du brevet: **10.01.90**

(51) Int. Cl.⁴: **C 07 C 19/08**, C 07 C 17/20

(21) Numéro de dépôt: **81402048.3**

(22) Date de dépôt: **22.12.81**

(54) **Procédé continu de préparation en phase gazeuse du trichlorotrifluoréthane, du dichlorotétrafluoréthane et du monochloropentafluoréthane en proportions contrôlées.**

(30) Priorité: **31.12.80 FR 8027865**

(43) Date de publication de la demande: **28.07.82 Bulletin 82/30**

(45) Mention de la délivrance du brevet: **08.08.84 Bulletin 84/32**

(45) Mention de la décision concernant l'opposition: **10.01.90 Bulletin 90/2**

(84) Etats contractants désignés: **BE DE FR GB IT NL**

(56) Documents cités:
DD-A-117 964
DD-A-119 033
DD-C-118 221
DE-A-1 568 617
DE-A-1 903 556
DE-B-2 137 499
FR-A-1 147 756
FR-A-1 166 211
FR-A-1 453 510
FR-A-2 000 688
US-A-2 744 147
US-A-2 755 313
US-A-3 157 707
US-A-3 413 363
US-A-3 632 834

(73) Titulaire: **ATOCHEM, 4 & 8, Cours Michelet La Défense 10, F-92800 Puteaux (FR)**

(72) Inventeur: **Abel, Michel, 7 Chemin du Perron, F-69310 Pierre-Bénite (FR)**
Inventeur: **Fine, François, Place de l'Eglise, F-69720 Saint-Laurent-de-Mure (FR)**
Inventeur: **Foulletier, Louis, 5 Chemin Croix-Berthet, F-69600 Oullins (FR)**
Inventeur: **Verot, Yvan, 20, rue du Prieuré, F-69130 Ecully (FR)**

(74) Mandataire: **Rochet, Michel et al, ATOCHEM Département Propriété Industrielle, F-92091 Paris la Défense Cédex 42 (FR)**

(56) Documents cités: (suite)
**Journal of Florine Chemistry,19 (1981/82),p.21-34**
**ULLMANNS Encyclopädie der technischen Chemie, Verfahrenstechnik II und Reaktionsapparatur, 4.Auflage,Band 3, p.328,1973**

EP 0 056 548 B2

**Description**

La présente invention concerne un procédé continu de préparation en phase gazeuse des trichloro-1,1,2-trifluoro-1,2,2,-éthane, dichloro-1,2-tétrafluoro-1,1,2,2-éthane et chloro-1-pentafluoro-1,1,2,2,2-éthane, permettant d'obtenir ces trois composés dans des proportions prédéterminées, à partir de tétrachloréthylène, de chlore et d'acide fluorhydrique.

Les réactions de fluoruration catalytique de l'hexachloréthane, formé in situ par addition de chlore sur le tétrachloréthylène, sont connues depuis longtemps.

Un procédé couramment utilisé consiste à opérer ces réactions en phase liquide, en présence d'un catalyseur à base d'halogénure d'antimoine. Ce procédé permet d'obtenir aisément le trichloro-1,1,2-trifluoro-1,2,2-éthane, mais l'obtention du dichloro-1,2-tétrafluoro-1,1,2,2-éthane requiert des températures de réaction élevées, ce qui entraîne une forte corrosion de l'appareillage. L'obtention du chloro-1-pentafluoro-1,1,2,2-éthane en quantités significatives est pratiquement impossible. De plus, l'activité des catalyseurs utilisés en phase liquide est très sensible aux impuretés des réactifs, ce qui implique des purifications poussées élevant le coût du procédé. Il se forme également de faibles quantités de pentahaloéthanes, difficiles à séparer.

D'autres procédés permettent de réaliser ces réactions de fluoruration en phase gazeuse, sur des catalyseurs divers à base d'oxydes ou d'halogénures de chrome, d'aluminium, de cobalt, de fer, de titane, de nickel, de cuivre, de palladium ou de zirconium, à des températures de l'ordre de 200 à 450 °C.

Ces procédés en phase gazeuse présentent eux aussi un certain nombre d'inconvénients. Le principal est la formation, plus importante en phase gazeuse qu'en phase liquide, de trichloro-1,1,1-trifluoro-2,2,2-éthane et de dichloro-1,1-tétrafluoro-1,2,2,2-éthane. Ces isomères, dits asymétriques, du trichloro-1,1,2-trifluoro-1,2,2-éthane et du dichlor-1,2-tétrafluoro-1,1,2,2-éthane, sont indésirables, car ils sont plus réactifs, et par suite plus instables, que les isomères dits symétriques. On admet que pour beaucoup d'applications commerciales le trichlorotrifluoréthane doit contenir moins de 2% de l'isomère trichloro-1,1,1-trifluoro-2,2,2-éthane; de même le dichlorotétrafluoréthane doit contenir moins de 15%, et de préférence moins de 7%, de l'isomère asymétrique dichloro-1,1-tétrafluoro-1,2,2,2-éthane.

Un autre inconvénient des procédés connus en phase gazeuse est la difficulté de contrôler la température de réaction, en raison de la forte exothermicité de la réaction d'addition du chlore sur le tétrachloréthylène.

Un troisième inconvénient provient de la désactivation du catalyseur, causée par la polymérisation du térachloréthylène sur la surface catalytique.

Enfin les procédés connus de fluoruration en phase gazeuse ne permettent que difficilement d'obtenir une répartition prédéterminée en trichlorotrifluoroéthane, dichlorotétrafluoréthane et monochloropentafluoréthane.

Pour pallier partiellement ces inconvénients, diverses solutions ont été préconisées. C'est ainsi que le brevet des Etats-Unis no 3 632 834 enseigne que l'utilisation d'un catalyseur spécifique à base de trifluorure de chrome permet d'obtenir en phase gazeuse du trichlorotrifluoréthane ne contenant pas plus d'environ 2% de l'isomère trichloro-1,1,1-trifluoro-2,2,2-éthane et du dichlorotétrafluoréthane ne contenant pas plus d'environ 15% de l'isomère dichloro-1,1-tétrafluoro-1,2,2,2-éthane. Mais ce procédé ne permet pas d'obtenir le chloropentafluoréthane et le taux de transformation de l'acide fluorhydrique ne dépasse pas 62%.

Le brevet des Etats-Unis no 3 157 707 décrit un procédé de préparation des tétrachlorodifluoréthane, trichlorotrifluoréthane, dichlorotétrafluoréthane et chloropentafluoréthane par passage en phase vapeur d'un mélange de tétrachloréthylène, de chlore et d'acide fluorhydrique sur un catalyseur à base de $Cr_2O_3$. Les isomères asymétriques sont produits en faibles quantités, non précisées. Un mode particulier de réalisation de ce procédé consiste à faire d'abord réagir, à une température de 200 à 350 °C, le chlore et le tétrachloréthylène sur un catalyseur à base de $Cr_2O_3$ puis à mélanger les effluents avec de l'acide fluorhydrique avant de les envoyer dans une deuxième zone de catalyse à l'oxyde de chrome, portée à une température de 300 °C. Cette variante ne permet d'obtenir que du trichlorotrifluoréthane det du dichlorotétrafluoréthane.

Pour l'obtention du trichlorotrifluoréthane, du dichlorotétrafluoréthane et du chloropentafluoréthane en proportions contrôlées, la demande de brevet japonais no 73.26 729 préconise une combinaison de réactions en phase liquide et en phase gazeuse. Dans un premeir temps on fait réagir le tétrachloréthylène, le chlore et l'acide fluorhydrique en phase liquide sous pression sur un catalyseur à base de trichlorure d'antimoine et on isole le trichlorotrifluoréthane brut formé. Ce dernier est alors envoyé en phase gazeuse, en présence d'acide fluorhydrique, sur un catalyseur à base de trifluorure d'aluminium. A 320 °C sous 5 atmosphères, 60% du trichlorotrifluoréthane sont convertis en dichlorotétrafluoréthane. Si la réaction est conduite à 440 °C sous 5 atmosphères, on obtient un rendement pratiquement quantitatif en chloropentafluoréthane. Ce procédé a pour principaux inconvénients une mauvaise utilisation de la chaleur de réaction entre le chlore et le tétrachloréthylène et l'obtention de quantités relativement importantes d'isomères asymétriques du trichlorotrifluoréthane et du dichlorotétrafluoréthane au cours de la fluoruration en phases gazeuse.

Le brevet no 118 221 de la République Démocratique Allemande décrit la préparation de chloropentafluoréthane en phase vapeur à partir

d'un mélange de tétrachloréthylène, de chlore et d'acide fluorhydrique. Ce mélange est d'abord envoyé dans une première zone de catalyse, chauffée à 340 °C et contenant un catalyseur au trifluorure d'aluminium dopé au fluorure de nickel; les effluents passent ensuite dans une deuxième zone de catalyse, contenant un catalyseur à l'oxyde de chrome chauffé à 350 °C. Il se forme comme sous-produits du trichlorotrifluoréthane, du dichlorotétrafluoréthane et de l'hexafluoréthane. Ce procédé a comme inconvénients majeurs d'utiliser deux catalyseurs différents, de ne pas permettre un contrôle facile des proportions des produits formés et de donner des teneurs très élevées en isomères asymétriques des $C_2Cl_3F_3$ et $C_2Cl_2F_4$.

Le brevet français no 1 453 510 de la demanderesse enseigne un procédé de purification du dichlorotétrafluoréthane, en vue de l'enrichir en isomère symétrique, qui consiste à faire passer, à l'état gazeux ou à l'état liquide, à des températures comprises entre 50 et 500 °C, le mélange d'isomères, éventuellement en présence d'acide fluorhydrique et/ou de chlore, sur un catalyseur choisi parmi le charbon actif, l'alumine activée, les tamis moléculaires, le fluorure d'aluminium, les sels de chrome, cobalt, aluminium, cuivre, fer ou molybdène, déposés ou non sur un support tel que le charbon actif ou l'alumine. Lorsqu'il est appliqué en présence d'acide fluorhydrique, ce procédé met en jeu deux réactions distinctes:

- une réaction de fluoruration du dichlorotétrafluoréthane, fournissant du chloropentafluoréthane

$$C_2Cl_2F_4 + HF \rightarrow C_2ClF_5 + HCl$$

avec formation accessoire de très faibles quantités d'hexafluoréthane,

- une réaction de dismutation du dichlorotétrafluoréthane, fournissant de chloropentafluoréthane et du trichlorotrifluoréthane

$$2C_2Cl_2F_4 \rightarrow C_2ClF_5 + C_2Cl_3F_3$$

L'isomère asymétrique du dichlorotétrafluoréthane étant, dans ces deux réactions, plus réactif que l'isomère symétrique, il en résulte un enrichissement du dichlorotétrafluoréthane, non tranformé, en isomère symétrique.

La demanderesse a maintenant découvert que, dans la préparation en phase gazeuse du trichlorotrifluorethane et du dichlorotétrafluoréthane à partir de chlore, d'acide fluorhydrique et de tétrachloréthylène:

- le taux de conversion de l'acide fluorhydrique est d'autant plus faible que le nombre d'atomes de fluor à fixer est plus élevé,
- plus le taux de conversion de l'acide fluorhydrique est faible, plus la teneur des produits de réactions en isomères asymétriques est faible,
- un excès de chlore permet de maintenir plus longtemps l'activité des catalyseurs de fluoruration.

La demanderesse a également découvert que, dans la préparation du chloropentafluoréthane et l'enrichissement du dichlorotétrafluoréthane en isomère symétrique selon le procédé du brevet français no 1 453 510, la présence dans les réactifs de trichlorotrifluoréthane et/ou d'acide chlorhydrique abaisse de façon appréciable le taux d'enrichissement du dichlorotétrafluoréthane en isomère symétrique. La présence d'acide chlorhydrique a de plus un rôle inhibteur sur la réaction de fluoruration du dichlorotétrafluoréthane en monochloropentafluoréthane.

Ces observations on conduit la demanderesse à concevoir un procédé catalytique continu de préparation en phase gazeuse du trichlorotrifluoréthane pauvre en isomère asymétrique, du dichlorotétrafluoréthane, pauvre également en isomère asymétrique, et du monochloropentafluoréthane, en proportions contrôlées, à partir de chlore, d'acide fluorhydrique et de tétrachloréthylène, en présence d'un catalyseur à base d'oxydes ou de sels de chrome, sous une pression de 0,1 à 20 bars, caractérisé par la combinaison des moyens suivants:

a) un premier réacteur de chloration-fluoruration, fonctionnant à une température comprise entre 300 et 500 °C, alimenté en chlore, acide fluorhydrique et produits de recyclage constitués de tétrachloréthylène, hexachloréthane, pentachlorofluoréthane, tétrachlorodifluoréthane et trichloritrifluoréthane, de façon à ce que le rapport molaire du chlore au tétrachloréthylène recyclé soit compris entre 1,1 et 5,0;

b) un deuxième réacteur de chloration-fluoruration, monté en série avec le premier, dont il reçoit les effluents additionnés de tétrachloréthylène frais de manière à ce que le rapport molaire global du chlore au tétrachloréthylène frais soit compris entre 0,95 et 1,05, et fonctionnant à une température compris entre 250 et 450 °C, toujours inférieure à la température de fonctionnement du premier réacteur;

c) un réacteur de fluoruration-dismutation, monté en parallèle avec les deux premiers réacteurs, fonctionnant à une température comprise entre 250 et 500 °C, et alimenté en acide fluorhydrique et en dichlorotétrafluoréthane recyclé, dans un rapport molaire choisi ente 0,1 et 1,0 en fonction de la proportion de chloropentafluoréthane désirée;

d) une unité de séparation permettant de traiter les gaz de réaction par des moyens connus pour récupérer l'acide fluorhydrique non consommé, extraire les quantités respectivement désirées de chloropentafluoréthane, de dichlorotétrafluoréthane et de trichlorotrifluoréthane, recycler au premier réacteur le tétrachloréthylène et l'hexachloréthane non transformés, le pentachlorofluoréthane, le tétrachlorodifluoréthane et l'excès de trichlorotrifluoréthane, et recycler au troisième réacteur l'excès de dichlorotétrafluoréthane.

Pour une meilleure compréhension du procédé selon l'invention, l'installation est représentée schématiquement sur la figure.

Le réacteur 1 est alimenté en 9 par un mélange

de chlore, provenant d'un stockage 5, d'acide fluorhydrique frais et récupéré, arrivant en 7 et provenant d'un stockage 6 et d'un recyclage 19, et de produits recyclés, provenant en 8 de l'unité de séparation et constitués de tétrachloréthylène, d'hexachloréthane, de pentachlorofluoréthane, de tétrachlorodifluoréthane et de trichlorotrifluoréthane. Le chlore introduit est en excès par rapport au tétrachloréthylène recyclé.

Les gaz sortant en 10 du réacteur 1 sont mélangés avec du tétrachloréthylène frais, provenant d'un stockage 4, de manière à ce que le rapport molaire global du chlore introduit en 9 au tétrachloréthylène frais soit compris entre 0,95 et 1,05.

Le mélange est envoyé par la conduite 11 à l'entrée du réacteur 2, travaillant à plus basse température que le réacteur 1. Les effluents du réacteur 2 vont par la canalisation 12 à l'unité de séparation. Ils sont constitués essentiellement de dichlorotétrafluoréthane, de trichlorotrifluoréthane, de tétrachlorodifluoréthane, de pentachlorofluoréthane, d'hexachloréthane, de tétrachloréthylène, d'acide fluorohydrique et d'acide chlorhydrique.

Le réacteur 3 est alimenté en 15 par un mélange d'acide fluorhydrique, amené par la canalisation 13 et de dichlorotétrafluoréthane recyclé, amené par la canalisation 14. Les effluents du réacteur 3 sont envoyés à l'unité de séparation par la tuyauterie 16.

A la sortie de l'unité de séparation, on recueille en 17 la quantité désirée de dichlorotétrafluoréthane; le reste est recyclé au réacteur 3 par la canalisation 14. Le dichlorotétrafluoréthane extrait contient moins de 7% d'isomère asymétrique. En 18 on extrait le chloropentafluoréthane. Par la conduite 19 on extrait la quantité désirée de trichlorotrifluoréthane, contenant moins de 2% d'isomère asymétrique; le reste est mélangé aux tétrachloréthylène, hexachloréthane, pentachlorofluoréthane et tétrachlorodifluoréthane sortant en 8 de l'unité de séparation.

L'acide fluorhydrique non transformé est recyclé par la conduite 20.

Les catalyseurs utilisés dans les réacteurs 1 et 2 peuvent être des catalyseurs de fluoruration connus, comme les oxydes ou halogénures de chrome, d'aluminium, de cobalt, de fer, de titane, de nickel, de cuivre, de palladium ou de zirconium, utilisés tels quels ou sur des supports. Il est cependant particulièrement avantageux d'utiliser les catalyseurs à base de microbilles d'oxyde de chrome, décrits dans la demande de brevet français no 8 027 659 au nom de la demanderesse, les catalyseurs à base de charbon actif imprégné de sulfate de chrome, décrits dans la demande de brevet français no 8 027 660 au nom de la demanderesse, les catalyseurs à base de charbon actif imprégné de trioxyde de chrome, décrits dans la demande de brevet français no 8 027 661 au nom de la demanderesse, et les catalyseurs à base de sels ou oxydes de chrome et de phosphate d'aluminium, décrits dans la demande de brevet français no 8 027 662 au nom de la demanderesse. Ces catalyseurs permettent déjà par eux-mêmes d'obtenir un taux élevé d'isomères symétriques du trichlorotrifluoréthane et du dichlorotétrafluoréthane.

Les catalyseurs utilisés dans le réacteur 3 pour la réaction de fluoruration-dismutation du dichlorotétrafluoréthane peuvent être les catalyseurs décrits dans le brevet français no 1 453 510, mais les catalyseurs cités dans les demandes de brevet français no 8 027 659, 8 027 660, 8 027 661 et 8 027 662 sus-mentionnées conviennent également.

Pour la facilité d l'exploitation du procédé, il est préféré d'employer le même catalyseur dans les trois réacteurs, mais ce n'est pas obligatoire.

Les catalyseurs de fluoruration et fluoruration-dismutation peuvent être utilisés en lit fixe, mais il est bien préférable de les utiliser en lit fluidisé, ce qui contribue encore à augmenter leur durée de vie. En effet ces catalyseurs s'empoisonnent par la formation de goudrons à leur surface. L'emploi d'un lit fluidisé provoque l'élimination de ces goudrons par la simple abrasion des grains frottant les uns sur les autres. La fluidisation permet également un contrôle très précis de la température de fonctionnement du catalyseur et une bonne homogénéité de cette température ce qui réduit la formation indésirée des dérivés perfluorés et aussi ds dérivés pentahalogénés résultant du craquage des molécules. Par ailleurs, l'utilisation de réacteurs à lit fluidisé donne la possibilité de maintenir constante l'activité du catalyseur, par renouvellement partiel régulier de celui-ci, sans interrompre le fonctionnement de l'installation.

Le procédé selon l'invention peut fonctionner à des pressions allant de 0,1 - 20 bars. Le choix de la pression de fonctionnement est fixé par des considérations technologiques et économiques.

Les températures de réaction peuvent aller de 250 à 500°C, la température du réacteur 1 étant toujours plus élevées que celle du réacteur 2.

Ces températures sont réglées en fonction de la répartition désirée des produits de réactions.

Les temps de contact des gaz avec le catalyseur peuvent varier de 1 à 20 secondes, également en fonction de la répartition désirée des produits de réaction.

Le rapport molaire du chlore au tétrachloréthylène est globalement très voisin de 1 (compris entre 0,95 et 1,05) mais à l'entrée du premier réacteur l'excès de chlore est compris entre 1,1 et 5.0.

Pour les deux premiers réacteurs, le rapport molaire de l'acide fluorhydrique au tétrachloréthylène est fonction de la répartition désirée en trichlorotrifluoréthane et dichlorotétrafluoréthane.

Pour le troisième réacteur, le rapport molaire de l'acide fluorhydrique au dichlorotétrafluoréthane peut varier de 0,1 à 1 suivant la quantité de chloropentafluoréthane désirée.

Les exemples suivants, donnés à titre non limitatif, illustrent divers aspects du procédé selon l'invention. Toutes les parties indiquées sont des parties en moles. Dans tous les exemples, le dichlorotétrafluoréthane extrait contient moins de 7% d'isomère asymétrique et le trichlorotrifluoréthane extrait contient moins de 2% d'isomère asymétrique.

Exemple 1

Les trois réacteurs de l'installation sont des réacteurs à lit fluidisé, garnis d'un même catalyseur à l'oxyde de chrome sous forme de microbilles, tel que décrit dans la demande de brevet français no 8 027 659 de la demanderesse.

La répartition désirée des produits de la réaction est:

| | |
|---|---|
| trichlorotrifluoréthane | 14% en moles |
| dichlorotétrafluoréthane | 70% en moles |
| chloropentafluoréthane | 16% en moles |

Pour cela, on choisit les conditions de marche suivantes:

Réacteur 1:

| | |
|---|---|
| température | 330 °C |
| pression | 2,0 atmosphères absolues |
| temps de séjour | 2,6 secondes |

Alimentation:

| | |
|---|---|
| chlore | 100 parties |
| acide fluorhydrique | 425 parties |
| $C_2Cl_4$ recyclé | 65 parties |
| $C_2Cl_6$, $C_2Cl_5F$ et $C_2Cl_4F_2$ recyclés | 35 parties |
| $C_2Cl_3F_3$ recyclé | 35 parties |

A la sortie le taux de transformation de l'acide fluorhydrique est de 75%.

Réacteur 2:

| | |
|---|---|
| température | 290 °C |
| pression | 1,5 atmosphère absolue |
| temps de séjour | 2,2 secondes |

Alimentation:

Effluents du premier réacteur $C_2Cl_4$ frais 100 parties

A la sortie, le taux gobal de transformation de l'acide fluorhydrique est passé à 91,7%. La partie organique des efluents contient:

| | |
|---|---|
| $C_2Cl_4$ non transformé | 65 parties |
| $C_2Cl_6$, $C_2Cl_5F$ et $C_2Cl_4F_2$ | 35 parties |
| $C_2Cl_3F_3$ | 45 parties |
| $C_2Cl_2F_4$ | 90 parties |

Après séparation des constituants dans l'unité de séparation, le dichlorotétrafluoréthane est renvoyé au réacteur 3 en même temps que 28 parties d'acide fluorhydrique.

Ce réacteur 3 est maintenu à 330 °C, sous une pression de 0,3 atmosphère absolue, et le temps de contact est de 5,6 secondes.

On obtient les produits suivants:

| | |
|---|---|
| $C_2Cl_3F_3$ | 4 parties |
| $C_2Cl_2F_4$ | 70 parties |
| $C_2ClF_5$ | 16 parties |

Tout le dichlorotétrafluoréthane et le chloropentafluoréthane sont extraits de l'installation, en même temps que 16 parties de trichlorotrifluoréthane. Le reste de trichlorotrifluoréthane est recyclé à l'entrée du réacteur 1 avec les autres produits moins fluorés.

Exemple 2

On utilise le même catalyseur que dans l'exemple 1, mais on désire obtenir une répartition différente des produits de la réaction, à savoir:

| | |
|---|---|
| $C_2Cl_3F_3$ | 23% en moles |
| $C_2Cl_2F_4$ | 68% en moles |
| $C_2ClF_5$ | 9% en moles |

Les conditions de marche des réacteurs sont les suivants:

Réacteur 1:

| | |
|---|---|
| température | 405 °C |
| pression | 1,9 atmosphère absolue |
| temps de séjour | 4,6 secondes |

Alimentation:

| | |
|---|---|
| $Cl_2$ | 100 parties |
| HF | 415 parties |
| $C_2Cl_4$recyclé | 28 parties |
| $C_2Cl_6$, $C_2Cl_5F$ et $C_2Cl_4F_2$ recyclés | 72 parties |
| $C_2Cl_3F_3$ recyclé | 35 parties |

A la sortie, le taux de transformation de l'acide fluorhydrique est de 56,6%.

Réacteur 2:

| | |
|---|---|
| température | 285 °C |
| pression | 1,4 atmosphère absolue |
| temps de séjour | 4,6 secondes |

Alimentation:

Effluents du réacteur 1, additionnés de 100 parties de $C_2Cl_4$ frais. A la sortie du réacteur 2, le taux de transformation global de l'acie fluorhydrique atteint 91,3%.

La partie organique des effluents a la composition suivante:

| | |
|---|---|
| $C_2Cl_4$ non transformé | 28 parties |
| $C_2Cl_6$, $C_2Cl_5F$ et $C_2Cl_4F_2$ | 72 parties |
| $C_2Cl_3F_3$ | 55 parties |
| $C_2Cl_2F_4$ | 80 parties |

Après séparation des produits de la réaction, le dichlorotétrafluoréthane est envoyé au réacteur 3 en même temps que 44 parties d'acid fluorhydrique. Ce réacteur 3 fonctionne à 395 °C, sous 0,7 atmosphère, avec un temps de séjour de 4,9 secondes. Il se forme:

| | |
|---|---|
| $C_2Cl_3F_3$ | 3 parties |
| $C_2Cl_2F_4$ | 68 parties |
| $C_2ClF_5$ | 9 parties |

On extrait de l'installationtout le chloropentafluoréthane, tout le dichlorotétrafluoréthane et 23 parties de trichlorotrifluoréthane. Le reste du $C_2Cl_3F_3$ est recyclé au premier réacteur avec les autres produits moins fluorés.

Exemple 3

Les trois réacteurs à lit fluidisé sont garnis d'un même catalyseur à l'oxyde de chrome déposé sur charbon actif, tel que décrit dans la demande de brevet français no 8 027 661 de la demanderesse.

Pour obtenir la réparation suivante des produits de la réaction:

| | |
|---|---|
| $C_2Cl_3F_3$ | 78,9% |
| $C_2Cl_2F_4$ | 17,3% |
| $C_2ClF_5$ | 3,8% |

on choisit les conditions de marche suivantes:

Réacteur 1:

| | |
|---|---|
| température | 375 °C |
| pression | 2,1 atmosphères absolues |
| temps de séjour | 7,3 secondes |

L'alimentation est faite avec le mélange suivant:

| | |
|---|---|
| $Cl_2$ | 100 parties |
| HF | 341 parties |
| $C_2Cl_4$ recyclé | 50 parties |
| $C_2Cl_6$, $C_2Cl_5F$ et $C_2Cl_4F_2$ recylcés | 50 parties |
| $C_2Cl_3F_3$ recyclé | 29 parties |

Le taux de transformation de l'acide fluorhydrique dans ce réacteur est de 68%.

Réacteur 2:

| | |
|---|---|
| température | 300 °C |
| pression | 1,2 atmosphère absolue |
| temps de séjour | 7,3 secondes |

L'alimentation est faite par les effluents du réacteur 1, additionnés de 100- parties de $C_2Cl_4$ frais. A la sortie, le taux de transformation global de l'acide fluorhydrique s'élève à 95%. La partie organique des effluents est constituée de:

| | |
|---|---|
| $C_2Cl_4$ non transformé | 50,0 parties |
| $C_2Cl_6$, $C_2Cl_5F$, $C_2Cl_4F_2$ | 50,0 parties |
| $C_2Cl_3F_3$ | 78,9 parties |
| $C_2Cl_2F_4$ | 24,0 parties |

Réacteur 3:

| | |
|---|---|
| température | 390 %C |
| pression | 1,5 atmosphère absolue |
| temps de séjour | 7,0 secondes |

Ce réacteur est alimenté par le dichlorotétrafluoréthane produit dans les deux premiers réacteurs, additionné de 2,5 parties d'acide fluorhydrique.

A la sortie de ce réacteur, on obtient:

| | |
|---|---|
| $C_2Cl_3F_3$ | 2,9 parties |
| $C_2Cl_2F_4$ | 17,3 parties |
| $C_2ClF_5$ | 3,8 parties |

Le dichlorotétrafluoréthane et le chloropentafluoréthane. L'excès non désiré de trichlorotrifluoréthane est recyclé au réacteur 1 avec les $C_2Cl_6$, $C_2Cl_5F$ et $C_2Cl_4F_2$.

Exemple 4

On opère avec le même catalyseur que dans l'exemple 3. Pour assurer une production de:

| | |
|---|---|
| $C_2Cl_2F_4$ | 6,5% |
| et $C_2ClF_5$ | 93,5% |

on utilise les conditions de marche suivantes:

Réacteur 1:

| | |
|---|---|
| température | 420 °C |
| pression | 2,6 atmosphères absolues |
| temps de séjour | 6,0 secondes |

L'alimentation est faite par le mélange suivant:

| | |
|---|---|
| chlore | 101,8 parties |
| acide fluorhydrique | 488,5 parties |
| $C_2Cl_4$ recyclé | 50,5 parties |
| $C_2Cl_6$, $C_2Cl_5F$ et $C_2Cl_4F_2$ recyclés | 44,4 parties |
| $C_2Cl_3F_3$ recyclé | 108,2 parties |

Le taux de transformation de l'acide fluorhydrique est de 65% dans ce réacteur.

Réacteur 2:

| | |
|---|---|
| température | 320 °C |
| pression | 2,1 atmosphères absolues |
| temps de séjour | 6,0 secondes |

L'alimentation est faite par les effluents du réacteur 1, additionnés de 100 parties de tétrachlorethylène frais.

A la sortie, le taux de transformation global de l'acide fluorhydrique est de 85%. Les effluents organiques sont composés de:

| | |
|---|---|
| $C_2Cl_4$ | 50,5 parties |
| $C_2Cl_6$, $C_2Cl_5F$, $C_2Cl_4F_2$ | 44,4 parties |
| $C_2Cl_3F_3$ | 93,8 parties |
| $C_2Cl_2F_4$ | 114,4 parties |

Réacteur 3:

| | |
|---|---|
| température | 420 °C |
| pression | 1,5 atmosphère absolue |
| temps de séjour | 10,2 secondes |

Le réacteur 3 est alimenté par 114,4 partis de $C_2Cl_2F_4$ provenant du réacteur 2 et 245,8 parties de $C_2Cl_2F_4$ recyclé, auxquelles on a ajouté 150,6 parties d'acide fluorhydrique.

A la sortie du réacteur, les effluents organiques contiennent:

| | |
|---|---|
| $C_2Cl_3F_3$ | 14,4 parties |
| $C_2Cl_2F_4$ | 252,1 parties |
| $C_2ClF_5$ | 90,0 parties |
| $C_2F_6$ | 3,6 parties |

On sort de l'installation tout le $C_2ClF_5$ et 6,3 parties de $C_2Cl_2F_4$. Le reste du $C_2Cl_2F_4$ est recyclé au réacteur 3, tandis que tout le $C_2Cl_3F_3$ produit est recyclé au réacteur 1.

Exemple 5

Dans les trois réacteurs à lit fluidisé on utilise un catalyseur au sulfate de chrome déposé sur charbon actif, tel que décrit dans la demande de brevet français no 8 027 660 de la demanderesse.

On veut obtenir la répartition suivante des produits de la réaction:

| | |
|---|---|
| $C_2Cl_3F_3$ | 13,2% |
| $C_2Cl_2F_4$ | 80,1% |
| $C_2ClF_5$ | 6,7% |

Les conditions de fonctionnement sont alors les suivantes:

Réacteur 1:

| | |
|---|---|
| température | 400 °C |
| pression | 2,1 atmosphère absolues |
| temps de séjour | 6,0 secondes |

Alimentation:

| | |
|---|---|
| $C_2$ | 100,0 parties |
| HF | 489,3 parties |
| $C_2Cl_4$ recyclé | 47,0 parties |
| $C_2Cl_6$, $C_2Cl_5F$ et $C_2Cl_4F_4$ recyclés | 35,0 parties |
| $C_2Cl_3F_3$ recyclé | 115,9 parties |

Le taux de transformation de l'acide fluorhydrique dans ce réacteur est de 54%.

Réacteur 2:

| | |
|---|---|
| température | 300 °C |
| pression | 1,6 atmosphère absolue |
| temps de séjour | 4,0 secondes |

Le réacteur est alimenté par les effluents du réacteur 1, additionnés de 100 parties de tétrachloréthylène frais.

Le taux de transformation global de l'acide fluorhydrique atteint 79% à la sortie de ce réacteur et la composition des effluents organiques est la suivante:

| | |
|---|---|
| $C_2Cl_4$ | 47,0 parties |
| $C_2Cl_6$, $C_2Cl_5F$ et $C_2Cl_4F_2$ | 35,0 parties |
| $C_2Cl_3F_3$ | 127,4 parties |
| $C_2Cl_2F_4$ | 88,5 parties |

Réacteur 3:

| | |
|---|---|
| température | 385 °C |
| pression | 1,5 atmosphère absolue |
| temps de séjour | 9 secondes |

Tout le dichlorotétrafluoréthane issu du réacteur 2 est envoyé dans le réacteur 3 en même temps que 12,8 parties d'HF.

Les effluents sont constitués de:

| | |
|---|---|
| $C_2Cl_3F_3$ | 1,7 parties |
| $C_2Cl_2F_4$ | 80,1 parties |
| $C_2ClF_5$ | 6,7 parties |

On extrait de l'installation la totalité du dichlorotétrafluoréthane et du chloropentafluoréthane, ainsi que 13,2 parties de trichlorotrifuloréthane. Le reste de ce produit est recyclé au réacteur 1.

Exemple 6

On opère comme dans l'exemple 5, mais avec les conditions de marche suivantes:

Réacteur 1:

| | |
|---|---|
| température | 375 °C |
| pression | 2,5 atmosphères absolues |
| temps de séjour | 3,6 secondes |

Alimentation:

| | |
|---|---|
| $Cl_2$ | 100 parties |
| HF | 444 parties |
| $C_2Cl_4$ recyclé | 71 parties |
| $C_2Cl_6$, $C_2Cl_5F$ et $C_2Cl_4F_2$ recyclés | 42 parties |
| $C_2Cl_3F_3$ recyclé | 129 parties |

Le taux de transformation de l'HF dans ce réacteur est de 74,5%.

Réacteur 2:

| | |
|---|---|
| température | 290 °C |
| pression | 2,0 atmosphères absolues |
| temps de séjour | 4,1 secondes |

Le réacteur est alimenté par les effluents du réacteur 1, additionnés de 100 parties de $C_2Cl_4$ frais.

Le taux de transformation global de l'HF dans les deux premiers réacteurs est de 87,6%;

La composition des effluents organiques est la suivante:

| | |
|---|---|
| $C_2Cl_4$ | 71 parties |
| $C_2Cl_6$, $C_2Cl_5F$, $C_2Cl_4F_2$ | 42 parties |
| $C_2Cl_3F_3$ | 140 parties |
| $C_2Cl_2F_4$ | 89 parties |

Réacteur 3:

| | |
|---|---|
| température | 400 °C |
| pression | 1,4 atmosphère absolue |
| temps de séjour | 8,9 secondes |

Ce réacteur est alimenté par 89 parties de $C_2Cl_2F_4$ et 42 parties d'HF. A la sortie, les effluents contiennent:

| | |
|---|---|
| $C_2Cl_3F_3$ | 5 parties |
| $C_2Cl_2F_4$ | 65 parties |
| $C_2ClF_5$ | 19 parties |

On extrait de l'installation tout le $C_2Cl_2F_4$ et toute le $C_2ClF_5$, ainsi que 16 parties de $C_2Cl_3F_3$. Le reste de $C_2Cl_3F_3$ est recyclé au réacteur 1 avec les autres produits sous-fluorés.

Exemple 7

Toujours avec le catalyseur de l'exemple 5 dans les trois réacteurs, on utilise les conditions suivantes:

Réacteur 1:

| | |
|---|---|
| température | 410 °C |
| pression | 2,6 atmosphères absolues |
| temps de séjour | 2,8 secondes |

Le réacteur est alimenté par un mélange de:

| | |
|---|---|
| chlore | 100 parties |
| acide fluorhydrique | 350 parties |
| $C_2Cl_4$ recyclé | 39 parties |
| $C_2Cl_6$, $C_2Cl_5F$, $C_2Cl_4F_2$ recyclés | 91 parties |
| $C_2Cl_3F_3$ recyclé | 74 parties |

Le taux de transformation de l'acie fluorhydrique à la sortie est de 60,8%.

Réacteur 2:

| | |
|---|---|
| température | 280 °C |
| pression | 2,0 atmosphères absolues |
| temps de séjour | 2,5 secondes |

Le réacteur est alimenté par les effluents du réacteur 1, additonnés de 100 parties de tétrachloréthylène frais.

Le taux global de transformation de l'acide fluorhydrique dans les deux premiers réacteurs est de 98,5%.

La composition des effluents organiques est la suivante:

| | |
|---|---|
| $C_2Cl_4$ | 39 parties |
| $C_2Cl_6$, $C_2Cl_5F$, $C_2Cl_4F_2$ | 91 parties |
| $C_2Cl_3F_3$ | 146 parties |
| $C_2Cl_2F_4$ | 28 parties |

Réacteur 3:

| | |
|---|---|
| température | 400 °C |
| pression | 1,3 atmosphère absolue |
| temps de séjour | 9,5 secondes |

Ce réacteur est alimenté par un mélange de 28 parties de $C_2Cl_2F_4$ et de 8 parties d'acide fluorhydrique.

A la sortie on obtient:

| | |
|---|---|
| $C_2Cl_3F_3$ | 1 partie |
| $C_2Cl_2F_4$ | 23 parties |
| $C_2ClF_5$ | 4 parties |

On extrait de l'installation tout le $C_2Cl_2F_4$, tout le $C_2ClF_5$ et 73 parties de $C_2Cl_3F_3$. Le reste de $C_2Cl_3F_3$ est recyclé au premier réacteur avec les autes produits moins fluorés.

Exemple 8

Les trois réacteurs à lit fluidisé sont remplis d'un catalyseur aux sels de chrome déposés sur phosphate d'aluminium, tel que décrit dans la demande de brevet franạis no 8 027 662 de la demanderesse. On désire produire uniquement 70% de $C_2Cl_2F_4$ et 30% de $C_2ClF_5$. Les conditions opératoires sont les suivantes:

Réacteur 1

| | |
|---|---|
| température | 411 °C |
| presion | 2,2 atmopshères absolues |
| temps de séjour | 4,6 secondes |

L'alimentation est faite par un mélange de:

| | |
|---|---|
| chlore | 100 parties |
| acide fluorhydrique | 433 parties |
| $C_2Cl_4$ recyclé | 32 parties |
| $C_2Cl_6$, $C_2Cl_5F$ et $C_2Cl_4F_2$ recyclés | 43 parties |
| $C_2Cl_3F_3$ recyclé | 91 parties |

Le taux de transformation de l'HF est de 56,9%.

Réacteur 2:

| | |
|---|---|
| température | 305 °C |
| pression | 1,9 atmosphère absolue |
| temps de séjour | 4,2 secondes |

Le réacteur est alimenté par les effluents du réacteur 1 mélangés à 100 parties de $C_2Cl_4$ frais. A la sortie, les gaz contiennent:

| | |
|---|---|
| $C_2Cl_4$ | 32 parties |
| $C_2Cl_6$, $C_2Cl_5F$ et $C_2Cl_4F_2$ | 43 parties |
| $C_2Cl_3F_3$ | 84 parties |
| $C_2Cl_2F_4$ | 106 parties |

Le taux global de transformation de l'acide fluorhydrique est de 92%.

Réacteur 3:

| | |
|---|---|
| température | 410 °C |
| pression | 0,9 atmopsphère absolue |
| temps de séjour | 5,6 secondes |

Le réacteur est alimenté par 106 parties de $C_2Cl_2F_4$ et 55 parties d'acide fluorhydrique. On obtient à la sortie:

| | |
|---|---|
| $C_2Cl_3F_3$ | 7 parties |
| $C_2Cl_2F_4$ | 70 parties |
| $C_2ClF_5$ | 29 parties |

Tout le $C_2Cl_3F_3$ produit est recyclé, tandis que l'on sort de l'installatoin la totalité du $C_2Cl_2F_4$ et du $C_2ClF_5$.

Exemple 9

On opère comme dans l'exemple 8, avec le même catalyseur, mois on cherche à obtenir la production suivante:

| | |
|---|---|
| $C_2Cl_3F_3$ | 47% |
| $C_2Cl_2F_4$ | 42% |
| $C_2ClF_5$ | 11% |

Les trois réacteurs fonctionnement alors dans les conditions suivantes:

Réacteur 1:

| | |
|---|---|
| température | 408 °C |
| pression | 1,8 atmosphère absolue |
| temps de séjour | 5,7 secondes |

On alimente le réacteur par un mélange de:

| | |
|---|---|
| chlore | 100 parties |
| acide fluorhydrique | 384 parties |
| $C_2Cl_4$ recyclé | 71 parties |
| $C_2Cl_6$, $C_2Cl_5F$, $C_2Cl_4F_2$ recyclés | 32 parties |
| $C_2Cl_3F_3$ recyclé | 79 parties |

Le taux de transformation de l'acide fluorhydrique est de 78,9%.

Réacteur 2:

| | |
|---|---|
| température | 290°C |
| pression | 1,1 atmosphère absolue |
| temps de séjour | 5,9 secondes |

Le réacteur est alimenté par les effluents du réacteur 1 additionnés de 100 parties $C_2Cl_4$ frais. Le taux global de transformation de l'acide fluorhydrique dans les deux réacteurs atteint 94%. Les gaz sortant sont composés de:

| | |
|---|---|
| $C_2Cl_4$ | 71 parties |
| $C_2Cl_6$, $C_2Cl_5F$, $C_2Cl_4F_2$ | 32 parties |
| $C_2Cl_3F_3$ | 122 parties |
| $C_2Cl_2F_4$ | 57 parties |

Réacteur 3:

| | |
|---|---|
| température | 410°C |
| pression | 0,9 atmosphère absolue |
| temps de séjour | 5,9 secondes |

Le réacteur est alimenté par un mélange de 57 parties de $C_2Cl_2F_4$ et de 18 parties d'acide fluorhydrique. On obtient à la sortie:

| | |
|---|---|
| $C_2Cl_3F_3$ | 4 parties |
| $C_2Cl_2F_4$ | 42 parties |
| $C_2ClF_5$ | 11 parties |

On extrait de l'installation tout le $C_2Cl_2F_4$, tout le $C_2ClF_5$ et 47 parties de $C_2Cl_3F_3$, le reste étant recyclé avec les produits moins fluorés au premier réacteur.

**Patentansprüche**

1. Kontinuierliches Verfahren zur Herstellung von Trichlortrifluorethan, Dichlortetrafluorethan und Monochlorpentafluorethan in kontrollierten Anteilen in der Gasphase ausgehend von Chlor, Fluorwasserstoffsäure und Tetrachlorethylen und in Gegenwart eines Katalysators auf Basis von Oxiden oder Salzen des Chroms bei einem Druck von 0,1 bis 20 bar, gekennzeichnet durch die Kombination der nachfolgenden Einrichtungen:

a) einem ersten Reaktor zur Chlorierung-Fluorierung der im Fliessbett bei einer Temperatur zwischen 300 und 500°C arbeitet und mit Chlor, Fluorwasserstoffsäure und Rückführungsprodukten, die aus Tetrachlorethylen, Hexachlorethan, Pentachlorfluorethan, Tetrachlordifluorethan und Trichlortrifluorethan bestehen, so gespeist wird, dass das molare Verhältnis des Chlors zum rückgeführten Tetrachlorethylen zwischen 1,1 und 5,0 liegt;

b) einem zweiten Chlorierungs-Fluorierungsreaktor, der zu dem ersten Reaktor in Serie angeordnet ist, dessen Produktstrom dieser aufnimmt, angereichert miti frischem Tetrachlorethylen, derart, dass das globale molare Verhältnis des Chlors zum frischen Tetrachlorethylen zwischen 0,95 und 1,05 liegt, der im Fliessbett bei einer Temperatur zwischen 250 und 450°C arbeitet, die immer unterhalb der Betriebstemperatur des ersten Reaktors liegt;

c) einem Fluorierungs-Dismutationsreaktor, der parallel zu den beiden ersten Reaktoren angeordnet ist, der bei einer Temperatur zwischen 250 und 500°C arbeitet und mit Fluorwasserstoffsäure und rückgeführtem Dichlortetrafluorethan gespeist wird, in einem molaren Verhältnis, das zwischen 0,1 und 1,0 gewählt wird, in Abhängigkeit von dem gewünschten Anteil an Chlorpentafluorethan;

d) einer Trenneinheit, die es ermöglicht, die Reaktionsgase in bekannter Weise zu behandeln, um die nichtverbrauchte Fluorwasserstoffsäure zurückzugewinnen, die jeweiligen gewünschten Mengen an Chlorpentafluorethan, Dichlortetrafluorethan und Trichlortrifluorethan zu extrahieren, das nicht umgewandelte Tetrachlorethylen und Hexachlorethan sowie Pentachlorfluorethan, Tetrachlordifluorethan und den Überschuss an Trichlortrifluorethan zum ersten Reaktor zurückzuführen und den Überschuss an Dichlortetrafluorethan zum dritten Reaktor zurückzuführen.

2. Verfahren nach Anspruch 1, dadurch gekennzeichnet, dass in den drei Reaktoren der gleichen Katalysator eingesetzt wird.

3. Verfahren nach Anspruch 1 oder 2, dadurch gekennzeichnet, dass der Reaktor der Fluorierung-Dismutation ein Reaktor mit Fliessbett ist.

4. Verfahren nach Anspruch 1 oder 2, dadurch gekennzeichnet, dass der Reaktor der Fluorierungs-Dismutation ein Reaktor mit Festbett ist.

**Claims**

1. A continuous process for the preparation in the gaseous phase of trichlorotrifluoroethane, dichlorotetrafluoroethane and monochloropentafluoroethane in controlled proportions from chlorine, hydrofluoric acid and tetrachloroethylene in the presence of a catalyst based on chromium oxides or salts at a pressure of 0.1 to 20 bars, characterized by the combination of the following means:

a) a first chlorination-fluoridation reactor operating as a fluidized bed at a temperature between 300 and 500°C and so supplied with chlorine, hydrdofluoric acid and recycling products formed by tetrachloroethylene, hexachloroethane, pentachlorofluoroethane, tetrachlorodifluoroethane and trichlorotrifluoroethane that the molar ratio between the chlorine and the recycled tetrachloroethylene is between 1.1 and 5.0;

b) a second chlorination-fluoridation reactor mounted in series with the first reactor, from

which it receives effluents with the addition of fresh tetrachloroethylene so that the total molar ratio between the chlorine and the fresh tetrachloroethylene is between 0,95 and 1.05, and operating as a fluidized bed at a temperature between 250 and 450°C, but in any case lower than the operating temperature of the first reactor;

c) a fluoridation-dismutation reactor mounted in parallel with the first two reactors, operating at a temperature between 250 and 500°C and supplied with hydrofluoric acid and recycled dichlorotetrafluoroethane in a molar ratin between 0.1 and 1.0 in dependence on the required proportion of chloropentafluorethane;

d) a separation unit enabling the reaction gases to be treated by known means to recover the unconsumed hydrofluoric acid, extract the required quantities of chloropentafluorethane, dichlorotetrafluorethane and trichlorotrifluoroethane respectively, recycle the unconverted tetrachloroethylene and hexachloroethane, the pentachlorofluorethane, the tetrachlorodifluoroethane and the surplus trichlorotrifluoroethane to the first reactor, and recycle the surplus dichlorotetrafluorethane to the third reactor.

2. A process according to claim 1, characterized in that the same catalyst is used in the three reactors.

3. A process according to claims 1 or 2, characterized in that the fluoridation-dismutation reactor is a fluidized bed reactor.

4. A process according to claims 1 or 2, characterized in that the fluoridation-dismutation reactor is a fixed bed reactor.

**Revendications**

1. Procédé continu de préparation en phase gazeuse du trichlorotrifluoréthane, du dichlorotétrafluoréthane et du monochloropentafluoréthane, en proportions contrôlées, à partir de chlore, d'acide fluorhydrique et de tétrachloréthylène, et en présence d'un catalyseur à base d'oxydes ou de sels de chrome, sous une pression de 0,1 à 20 bars, caractérisé par la combinaison des moyens suivants:

a) un premier réacteur de chloration-fluoruration, fonctionnant en lit fluidisé à une température comprise entre 300 et 500°C, alimenté en chlore, acide fluorhydrique et produits de recyclage constitués de tétrachloréthylène, hexachloréthane, pentachlorofluoréthane, tétrachlorodifluoréthane et trichlorotrifluoréthane, de façon à ce que le rapport molaire du chlore au tétrachloréthylène recyclé soit compris entre 1,1 et 5,0;

b) un deuxième réacteur de chloration-fluoruration, monté en série avec le premier, dont il reçoit les effluents additionnés de tétrachloréthylène frais de manière à ce que le rapport molaire global du chlore au tétrachloréthylène frais soit compris entre 0,95 et 1,05, et fonctionnant en lit fluidisé à une température comprise entre 250 et 450°C, toujours inférieure à la température de fonctionnement du premier réacteur;

c) un réacteur de fluoruration-dismutation, monté en parallèle avec les deux premiers réacteurs, fonctionnant à une température comprise entre 250 et 500°C, et alimenté en acide fluorhydrique et en dichlorotétrafluoréthane recyclé, dans un rapport molaire choisi entre 0,1 et 1,0 en fonction de la proportion de chloropentafluoréthane désirée;

d) une unité de séparation permettant de traiter les gaz de réaction par des moyens connus pour récupérer l'acide fluorhydrique non consommé, extraire les quantités respectivement désirées de chloropentafluoréthane, de dichlorotétrafluoréthane et de trichlorotrifluoréthane, recycler au premier réacteur le tétrachloréthylène et l'hexachloréthane non transformés, le pentachlorofluoréthane, le tétrachlorodifluoréthane et l'excès de trichlorotrifluoréthane, et recycler au troisième réacteur l'excès de dichlorotétrafluoréthane.

2. Procédé selon la revendication 1, caractérisé en ce que le même catalyseur est utilisé dans les trois réacteurs.

3. Procédé selon la revendication 1 ou 2, caractérisé en ce que le réacteur de fluoruration-dismutation est un réacteur à lit fluidisé.

4. Procédé selon la revendication 1 ou 2, caractérisé en ce que le réacteur de fluoruration-dismutation est un réacteur à lit fixe.

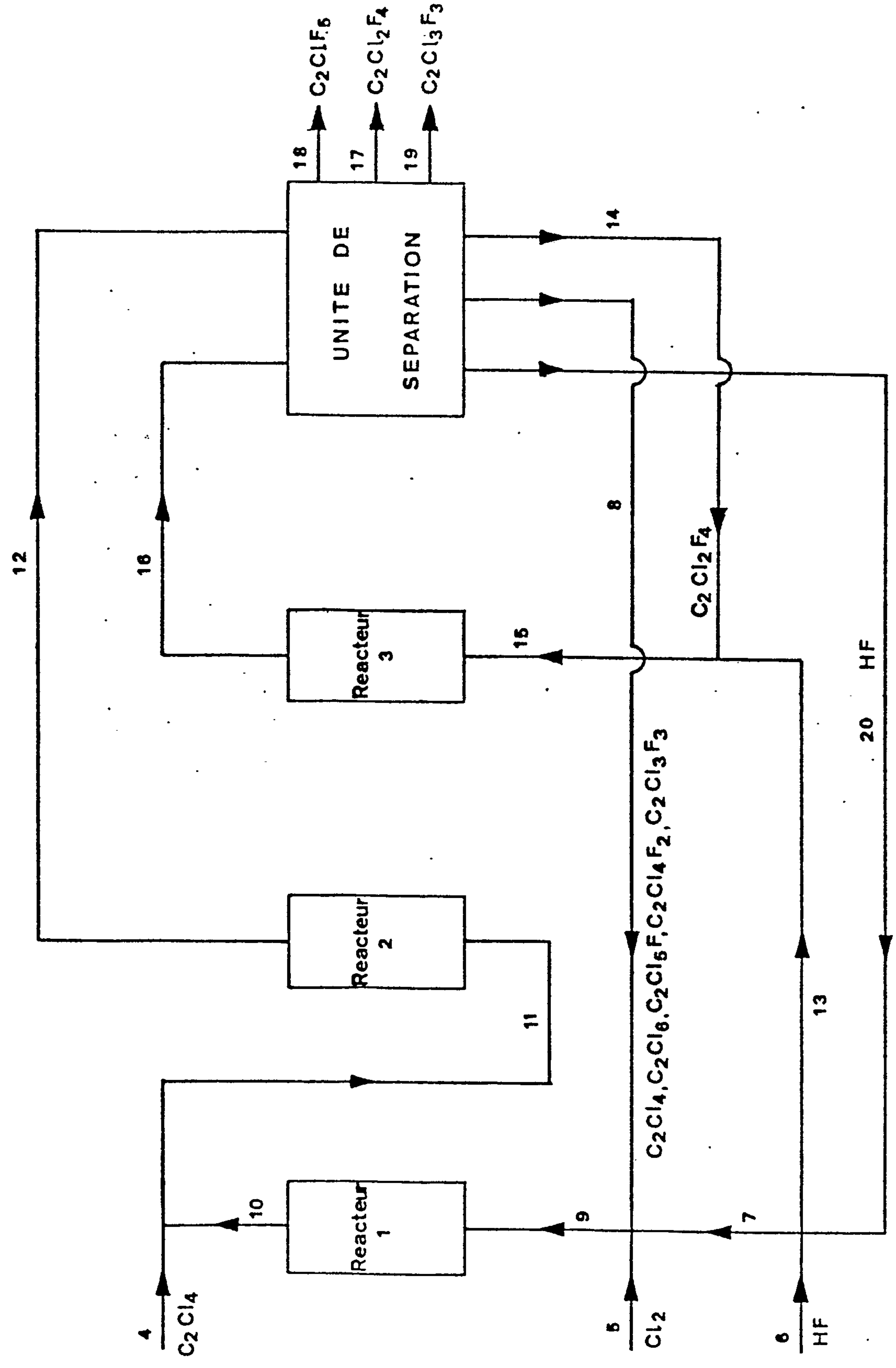
UNITE DE SEPARATION
Reacteur 1
Reacteur 2
Reacteur 3
$C_2ClF_5$
$C_2Cl_2F_4$
$C_2Cl_3F_3$
$C_2Cl_2F_4$
HF
$C_2Cl_4$, $C_2Cl_6$, $C_2Cl_5F$, $C_2Cl_4F_2$, $C_2Cl_3F_3$
$C_2Cl_4$
$Cl_2$
HF
4
5
6
7
8
9
10
11
12
13
14
15
16
17
18
19
20